(19) Europäisches Patentamt
European Patent Office
Office européen des brevets

(11) **EP 2 716 219 B1**

(12) **EUROPEAN PATENT SPECIFICATION**

(45) Date of publication and mention
of the grant of the patent:
**08.03.2017 Bulletin 2017/10**

(51) Int Cl.:
*A61B 5/20* (2006.01)    *G01F 23/26* (2006.01)
*G01F 1/00* (2006.01)

(21) Application number: **12792117.9**

(22) Date of filing: **22.05.2012**

(86) International application number:
**PCT/ES2012/070364**

(87) International publication number:
**WO 2012/164123 (06.12.2012 Gazette 2012/49)**

(54) **DEVICE FOR AUTOMATICALLY MEASURING THE AMOUNT OF FLOWING LIQUID AND THE METHOD FOR MEASURING THE LATTER**

VORRICHTUNG ZUR AUTOMATISCHEN MESSUNG DER MENGE EINER STRÖMENDEN FLÜSSIGKEIT UND VERFAHREN ZUR MESSUNG DIESER MENGE

DISPOSITIF DE MESURE AUTOMATIQUE DE LA QUANTITÉ DE LIQUIDE S'ÉCOULANT ET PROCÉDÉ DE MESURE CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2011 ES 201130898**

(43) Date of publication of application:
**09.04.2014 Bulletin 2014/15**

(73) Proprietors:
• **Consejo Superior De Investigaciones Científicas (CSIC)**
**28006 Madrid (ES)**
• **Universidad Politécnica De Madrid**
**28040 Madrid (ES)**
• **Universidad Ceu San Pablo De Madrid**
**28040 Madrid (ES)**

(72) Inventors:
• **FERNÁNDEZ SAAVEDRA, Roemi Emilia**
**E-28500 Arganda del Rey (Madrid) (ES)**
• **APALKOV, Andrey**
**E-28500 Arganda del Rey (Madrid) (ES)**
• **ARMADA RODRÍGUEZ, Manuel Ángel**
**E-28500 Arganda del Rey (Madrid) (ES)**
• **OTERO QUINTANA, Abraham**
**E-28040 Madrid (ES)**
• **PALACIOS ORTEGA, Francisco**
**E-28905 Getafe (Madrid) (ES)**

(74) Representative: **Ungria López, Javier**
**Avda. Ramón y Cajal, 78**
**28043 Madrid (ES)**

(56) References cited:
WO-A1-87/01025    WO-A1-96/08219
WO-A1-2010/149708    WO-A1-2010/149708
DE-A1- 3 923 079    ES-A1- 2 354 794
FR-A1- 2 289 165    GB-A- 1 574 066
US-A- 4 291 706    US-A- 4 305 405
US-A- 5 119 675    US-A- 5 409 014
US-A- 6 125 696    US-A- 6 125 696
US-A1- 2010 137 743    US-B1- 6 337 959
US-E- R E26 964

• ABRAHAM OTERO ET AL: "A Low Cost Device for Monitoring the Urine Output of Critical Care Patients", SENSORS, vol. 10, no. 12, 2 December 2010 (2010-12-02), pages 10714-10732, XP055103166, ISSN: 1424-8220, DOI: 10.3390/s101210714
• A. Otero ET AL: "A Prototype Device to Measure and Supervise Urine Output of Critical Patients" In: "Recent Advances in Biomedical Engineering", 1 October 2009 (2009-10-01), InTech, XP055104863, ISBN: 978-9-53-307004-9 pages 321-334, DOI: 10.5772/7474, * par. 2.1 The sensor * * par. 2.2 Actuator design * * par. 2.3 The microcontroller *

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- OTERO ET AL.: 'An add-on solution to take measures automatically from critical care urine meters' INTELLIGENT SIGNAL PROCESSING (WISP), 2011 IEEE 7TH INTERNATIONAL SYMPOSIUM 19 September 2011 - 21 September 2011,
- OTERO ET AL.: 'A device for automatically measuring and supervising the critical care patient's urine output' SENSORS vol. 10, no. 1, 26 January 2010, ISSN 1424-8220 pages 934 - 951
- UNOMEDICAL: 'UNOMETER 500. (AP-008203-MM).', [Online] 2010, Retrieved from the Internet: <URL:http://marketport.unomedical.com/marketingZone/images/download/11535-Unometer500 _ 400-lav-9648.pdf>
- OTERO ET AL.: 'A low cost device for monitoring the urine output of critical care patients' SENSORS vol. 10, no. 12, 02 December 2010, ISSN 1424-8220 pages 10714 - 10732
- OTERO ET AL.: 'A prototype device to measure and supervise urine output of critical patients' RECENT ADVANCES IN BIOMEDICAL ENGINEERING (INTECH) 01 October 2009, pages 321 - 334

**Description**

**OBJECT OF THE INVENTION**

The invention belongs to the field of medicine and can be used particularly for automation of liquid measurements which are introduced into or coming out from a patient.

**BACKGROUND OF THE INVENTION**

Some known technical solutions (patent EP-0 008 450, patent EP-0 471 413) envisage a collecting transparent recipient for urine having a scale by means of which it is possible to check the total volume collected. In these solutions, the verification and registration of the increase of urine volume should be performed by the nursing staff periodically.

In patent DE-32 40 191 ultrasonic sensors are used to register the level reached by the fluid in the urine collecting device. This technique is expensive and unreliable, as it should be ensured that the apparatus does not swing, which is a requirement difficult to achieve in intensive care units.

Patent DE-40 23 336 envisages an apparatus for urine control in which, according to the capacity, the filling volume of urine accumulated in a measuring chamber is measured, wherein the level of urine affects the ability of a measuring capacitor. In this technique, the assembly is suitable only for a single-use part, which is rather costly. Furthermore, to achieve long-term use of the system, it is necessary to regularly clean the chamber and the measuring capacitor, as otherwise the formation of sediments can lead to false results.

U.S. Patent 4,745,929 envisages an apparatus in which the level of fluid reached in a column of urine is recorded through a series of optical barriers that overlap in a staggered manner. This device requires a system of valves actuated by means of electromagnets, which involves high energy consumption.

Patent DE- 35 44 031 describes a device intended for measuring the weight of liquid (urine), whereas patent DE- 43 38 687, envisages an urinometer provided with two angle sensors that are used to compensate for the inclination of its housing, thereby correcting the error of the force measurement due to the weight. These technical solutions have the disadvantage of having a rather complex embodiment, which increases the construction costs, hampering the manufacture of these measuring devices for their daily operation in an intensive care unit.

Under the title of "A low cost device for monitoring the urine output of critical care patients" (SENSORS, vol. 10, no.12, 2 December 2010, pages 10714-10732, ISSN: 1424-8220,DOI:10.3390/s101210714), A. OTERO ET AL. disclose a device capable of automatically sensing and supervising the urine output of critical patients. The device comprises two containers of different volumes equipped with reed switches that are activated by a magnet located inside the containers. Under the title of "A Prototype Device to Measure and Supervise Urine Output of Critical Patients" (Recent Advances in Biomedical Engineering, 1 October 2009, InTech, ISBN: 978-9-53-307004-9 pages 321-334, DOI:10.5772/7474), A. OTERO ET AL. disclose a device capable of automatic monitoring and supervising the urine output of critical care unit patients. The device comprises two containers of different volumes and a microcontroller for monitoring the level of produced urine.

**DESCRIPTION OF THE INVENTION**

1. Brief description of the invention.

The invention is an apparatus and a method as set out in the claims appended hereto.

2. Detailed Description of the Invention

The present invention is related to a device for measuring the amount of liquid that flows, for example, the amount of urine excreted by a patient. Its simple design greatly reduces the number of parts required for its manufacture, and significantly reduces the costs of those single-use parts that must be replaced for each new patient. Furthermore, its simplicity facilitates both its use and its preparation in hospitals from widely available industrial elements. Although the device described here has been designed primarily for automatic monitoring of the flow of urine of a patient having a catheter, its application to other liquids, such as blood, is not ruled out.

The device is composed of a wood, plastic or metal support (1) in which a plastic liquid recipient (2) is installed parallel to the ground, the interior of which is divided into two chambers that communicate through the top, a first chamber (4) of small size suitable for the measurements in which a high resolution is desired, and a second larger chamber (5) for measurements that do not require high resolution, an external valve (6) at the bottom of the liquid recipient (2) which allows simultaneously evacuating the first chamber (4) and the second chamber (5) of the liquid recipient (2), a flexible tube (7) (made of, for example, plastic polymer) that allows the entry of liquid from the source (for example, a patient having a catheter) by the top of the first chamber (4) of the liquid recipient (2), a drainage pipe (8) (made of, for example, plastic polymer) connected to the external valve (6), an electronic unit (9) and, linked to it, a calculation unit (10). The liquid recipient (2) may have, for example, plastic tabs, by way of flanges, for attachment to the support (1). The liquid recipient (2) may be constructed such that the first chamber (4) and the second chamber (5) are adjacent and communicate through at least one opening (3) located at the top of said chambers. The liquid recipient (2) may also be constructed so that the first chamber (4) is contained within the second chamber (5) and they communicate via at least one opening (3) located at the top of the first chamber (4). Moreover, the device for automatically measuring the amount of flowing liquid has two noninvasive capacitive sensors (11, 12) installed (for example, adhered) on the outer wall of the liquid recipient (2), such that the longitudinal area of the first capacitive sensor (11) spans the full height of the first chamber (4) and the longitudinal area of the second capacitive sensor (12) spans the full height of the second chamber (5) and both the first capacitive sensor (11) and the second capacitive sensor (12) are connected to the electronic unit (9), to which they continuously send the electric signal generated indicative of the level of liquid contained in each of the chambers of the liquid recipient (2). The noninvasive feature of the capacitive sensors (11, 12) allows carrying out the measurements of the liquid level in each of the chambers without contact between the sensor and the fluid, and this eliminates the problems of sterilization. The electronic unit (9) consists of the circuitry necessary to convert the physical quantity measured in a digitized value. For the calculation unit (10), for example, a personal computer or a microcontroller can be used. The technical solution described has a simple design that significantly reduces the number of single-use parts that have to be replaced with each new patient, reducing costs and facilitating its use.

The liquid recipient (2) may have, on its external wall, two holders (13) made by way of sleeves which allow installing both the first capacitive sensor (11) and the second capacitive sensor (12) by simply inserting the same inside the holders (13). This embodiment facilitates the installation process of the capacitive sensors (11, 12), and enables the reuse of the same when it is necessary to replace the liquid recipient (2). The holders (13) can be manufactured, for example, with the same material used for manufacturing the liquid recipient (2).

The device for automatically measuring the amount of flowing liquid may contain a base (14) made of wood, plastic or metal attached to the support (1), for example, by means of screws, in which the capacitive sensors (11, 12) are fixed. This embodiment allows easily assembling and disassembling the liquid recipient (2) on the support (1) and reusing the capacitive sensors (11, 12) since said capacitive sensors (11, 12) would be attached to the base (14) when the liquid recipient (2) is disassembled.

The device for automatically measuring the amount of flowing liquid may contain an adjustable and removable frame (15) made of metal or plastic, which allows the configuration of the assembly of the capacitive sensors (11, 12) on the outer wall of the liquid recipient (2). This adjustable and removable frame (15) can be made from at least four rigid elements the length of which can be varied (16), for example toothed telescopic guides the ends of which are joined by brackets (17), and in such a way that the capacitive sensors (11, 12) are installed, by attachment elements (18) (for example, screws or pins), on at least one of these rigid elements the length of which can be varied (16). This embodiment allows both the first capacitive sensor (11) and the second capacitive sensor (12) to be reused even with different models of liquid recipient (2).

The external valve (6) can be provided with an actuator, for example an electric motor or an electromagnet, electrically connected to the electronic unit (9), which automatically controls its opening and closing.

The device for automatically measuring the amount of flowing liquid can have a liquid collection bag (19) made of a thermoplastic polymer, such as polyvinyl chloride. This liquid collection bag (19) is connected to the drainage pipe (8) and is situated at a level lower than the liquid recipient (2), and its volume is n times higher than the volume of the liquid recipient (2), where n is an integer greater than one. Due to its low manufacturing cost, the liquid collection bag (19) need not be reused, so it can be discarded after each use.

The connection between the electronic unit (9) and the calculation unit (10) can be done wirelessly, for example via Bluetooth or Wi-Fi. This embodiment, by eliminating the cables, gives autonomy to the device, and allows it to be easily transported together with the patient.

Functional description of the device

Initially, the connection of the flexible tube (7) with the source of liquid is carried out, for example, a patient having a catheter in the intensive care unit and whose volume of urine excreted on a determined time interval we want to monitor. Next, the characteristics of the liquid recipient (2) to be used, for example, the dimensions of the first chamber (4) and the second chamber (5) are selected in the calculation unit (10). With this information, the calculation unit (10) can adjust the measurement field of the first capacitive sensor (11) and of the second capacitive sensor (12) and determine the values $R_1$ and $R_2$ corresponding to the maximum volumes of liquid that the first chamber (4) and the second chamber (5) may contain, respectively. Consecutively, a first cycle begins in which the measurement $i$ of the volume of liquid $V_i$ contained in the first chamber (4) of the liquid recipient (2) with a predetermined time interval $T_1$ is carried out, using for this the first capacitive sensor (11) and the electronic unit (9). The value $V_i$ is transmitted from the electronic unit (9) to the calculation unit (10), where it is stored. This transmission can be performed serially RS-232C or wirelessly (Bluetooth or Wi-Fi). In the calculation unit (10) the relative flow of the liquid that has flowed is calculated in accordance with the following formula:

$$Q_i = \frac{V_i - V_{i-1}}{T_1} \qquad \text{for } i = 1, 2, 3, \ldots$$

Simultaneously, the calculation unit (10) is responsible for verifying that the calculated value $Q_i(t)$, is lower than the upper limit value $Q_u$ and higher than the lower limit value $Q_D$. Limit values $Q_D$ and $Q_u$ are defined in advance, before starting the process. If at any time the calculation unit (10) verifies that the condition $Q_D < Q_i(t) < Q_u$ is not being met, a warning signal is generated for the operator, for example, a visual and audible warning. If at the time when $V_i \geq R_1 - \varepsilon$, where $\varepsilon$ is a predetermined small value, the total time required to have reached said volume of liquid in the first chamber (4) exceeds a predetermined value $L_1$, it is proceeded to opening the external valve (6). This opening can be done manually, generating for this an audible warning to alert a human operator, or automatically using an actuator, for example, an electromagnet or an electric motor. The external valve (6) will remain open for a predetermined time $T_{v1}$, which will allow emptying the liquid recipient (2). After $T_{v1}$ has passed, the external valve (6) is closed (manually or automatically) and the measurement cycle begins again.

If at the time when $V_i \geq R_1 - \varepsilon$, the total time required to have reached said volume of liquid in the first chamber (4) is equal to or less than a predetermined value $L_1$, it means that liquid production is high, therefore, high resolution in measuring the amount of flowing liquid is not required. Therefore, and in order to avoid frequent openings of the external valve (6), which could cause considerable stress to said valve if the fluid production rate is very high, in addition to being a very heavy task in the case of being performed by a human operator, the liquid will be allowed to fill the first chamber (4) of the liquid recipient (2), whereby the liquid will begin to move from the first chamber (4) to the second chamber (5) by means of the opening (3) communicating said chambers and a second cycle will begin in which the measurement $i$ of the volume of liquid $W_i$ contained in the second chamber (5) of the liquid recipient (2) will be carried out with a predetermined time interval $T_2$, using for this the second capacitive sensor (12) and the electronic unit (9). The value $W_i$ is transmitted from the electronic unit (9) to the calculation unit (10), where it is stored. Then, in the calculation unit (10) the relative flow of the liquid that has flowed is calculated in the calculation unit (10) in accordance with the following formula:

$$S_i = \frac{W_i - W_{i-1}}{T_2} \qquad \text{for } i = 1, 2, 3, \ldots$$

Simultaneously, the calculation unit (10) is responsible for verifying that the calculated value $S_i(t)$, is lower than the upper limit value $S_u$ and higher than the lower limit value $S_D$. Limit values $S_D$ and $S_u$ are defined in advance, before starting the process. If at any time the calculation unit (10) verifies that the condition $S_D < S_i(t) < S_u$ is not being met, a warning signal is generated for the operator, for example, a visual and audible warning. The process stops at the point at which $W_i > R_2 - \mathbf{E}$, where $\mathbf{E}$ is a predetermined small value, or when the critical condition $S_i(t) < S_D$ occurs. Next the external valve (6) is opened. This opening can be done manually or automatically by means of an actuator, for example, an electromagnet or an electric motor. The external valve (6) will remain open for a predetermined time $T_{v2}$, which will allow emptying the liquid recipient (2). After $T_{v2}$ has passed, the external valve (6) is closed (manually or automatically) and the first cycle begins again.

**DESCRIPTION OF THE DRAWINGS**

For a better understanding of what is written in this specification, some drawings are accompanied in which, only by way of example, practical cases of embodiments of the device are represented.

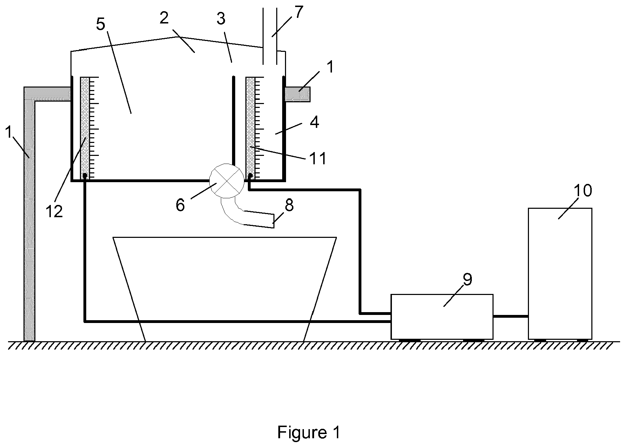

Figure 1 shows the configuration of the device for automatically measuring the amount of flowing liquid. The device is composed by a support (1) from which a liquid recipient (2) hangs, the interior of which is divided into two chambers, a first chamber (4) of small size suitable for the measurements where a high resolution is desired and a second larger chamber (5) for measurements that do not require high resolution, an external valve (6) at the bottom of the liquid recipient (2) which allows simultaneously evacuating the first chamber (4) and the second chamber (5) of the liquid recipient (2), a flexible tube (7) that allows the entry of liquid by the top of the first chamber (4) of the liquid recipient (2), a drainage pipe (8) connected to the external valve (6), an electronic unit (9) and, linked to it, a calculation unit (10). The liquid recipient (2) is constructed in such a way that the first chamber (4) and the second chamber (5) are adjacent and communicate through an opening (3) located at the top of the wall they share. Moreover, the device for automatically measuring the amount of flowing liquid has two capacitive sensors (11, 12) installed on the outer wall of the liquid recipient (2), such that the longitudinal area of the first capacitive sensor (11) spans the full height of the first chamber (4) and the longitudinal area of the second capacitive sensor (12) spans the full height of the second chamber (5) and both the first capacitive sensor (11) and the second capacitive sensor (12) are connected to the electronic unit (9).

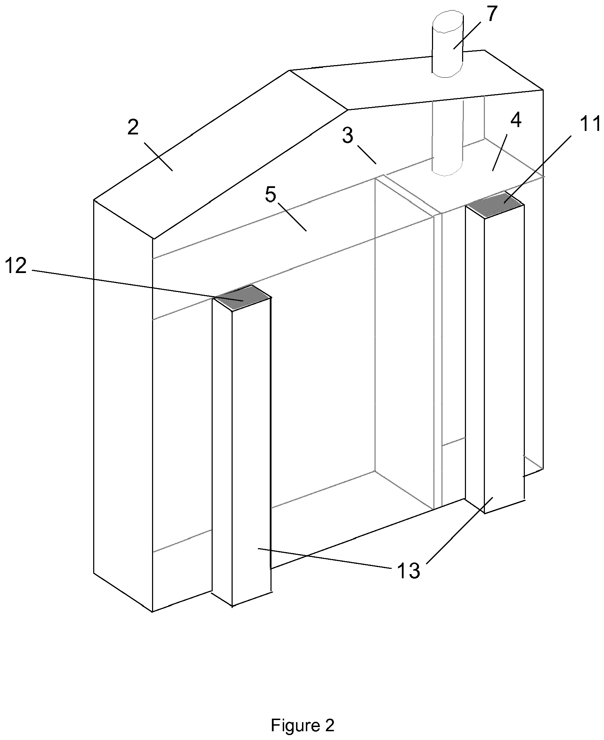

Figure 2 shows an embodiment of the liquid recipient (2), wherein it has on its outer wall, two holders (13) made by way of sleeves which allow installing both the first capacitive sensor (11) and the second capacitive sensor (12) by simply inserting the same inside the holders (13).

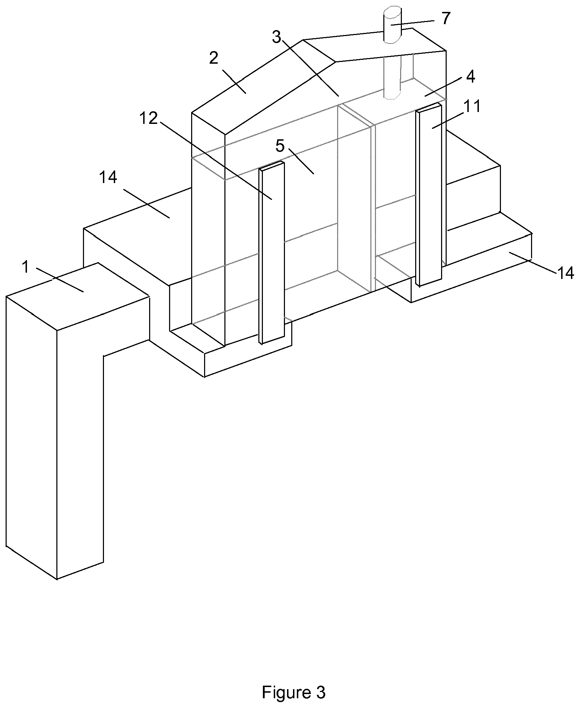

Figure 3 shows another embodiment of the device for automatically measuring the amount of flowing liquid containing a base (14) connected to the support (1), to which the capacitive sensors (11, 12) are attached and which allows to install the liquid recipient (2) in the support (1).

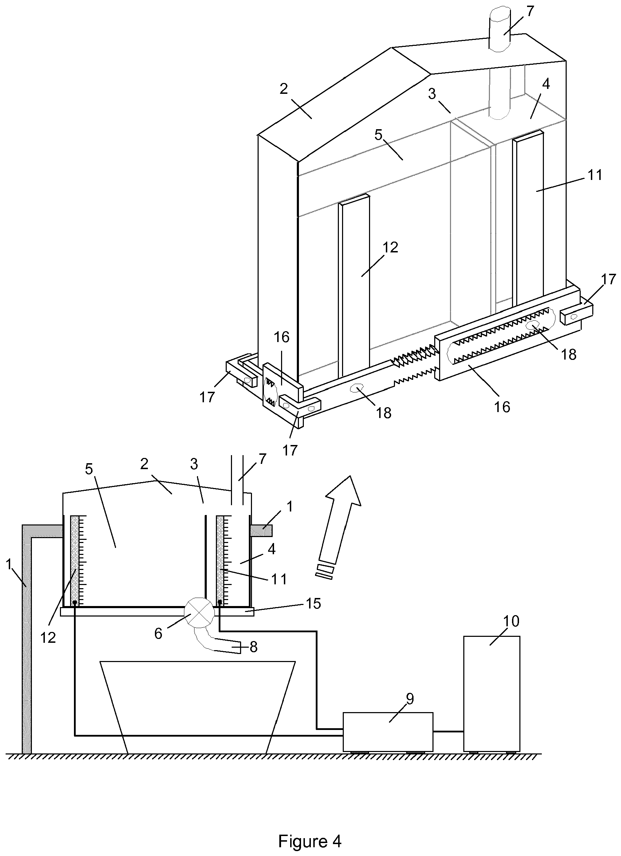

Figure 4 shows an embodiment of the device for automatically measuring the amount of flowing liquid containing an adjustable and removable frame (15) for configuring the attachment of capacitive sensors (11, 12) on the outer wall of the liquid recipient (2). This adjustable and removable frame (15) is made from four rigid elements the length of which can be varied (16), the ends of which are joined by brackets (17), and in such a way that the capacitive sensors (11, 12) are installed on one of these rigid elements the length of which can be varied (16), by means of attachment elements (18).

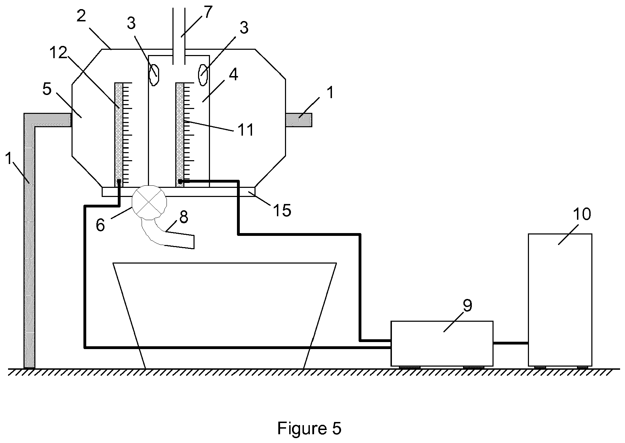

Figure 5 refers to an embodiment of the device for automatically measuring the amount of flowing liquid wherein the liquid recipient (2) is constructed in such a way that the first chamber (4) is contained within the second chamber (5) and communication between the two chambers is carried out by means of two openings (3) located on the top part of the first chamber (4).

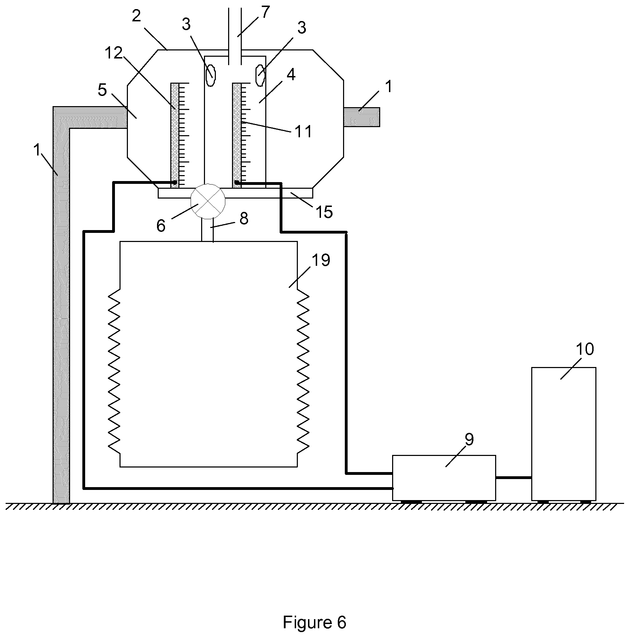

Figure 6 refers to an embodiment of the device wherein there is a liquid collection bag (19) connected to the drainage pipe (8) and located at a level lower than the liquid recipient (2) and the volume of which is n times higher than the volume of the liquid recipient (2), *n* being an integer greater than one.

List of designations

1. Support
2. Liquid recipient
3. Opening
4. First chamber
5. Second Chamber
6. External valve
7. Flexible tube
8. Drainage pipe
9. Electronic unit
10. Calculation unit
11. First capacitive sensor
12. Second capacitive sensor
13. Holders
14. Base
15. Adjustable and removable frame
16. Rigid elements the length of which can be varied
17. Brackets
18. Attachment elements
19. Collection bag

**PREFERRED EMBODIMENT OF THE INVENTION**

The present invention is further illustrated by the following example, which does not intend to limit its scope.

Example 1

The device for automatically measuring the amount of urine excreted is composed by a plastic liquid recipient (2) hanging from the patient's bed (which acts as a support (1)) and the interior of which is divided into two chambers; a first chamber (4) of small size suitable for the measurements where a high resolution is desired and a second larger chamber (5) for measurements that do not require high resolution, an external valve (6) at the bottom of the liquid recipient (2) which allows simultaneously evacuating the first chamber (4) and the second chamber (5) of the liquid recipient (2), an elbow free flexible tube (7) made of transparent, plastic polymer and with a length of 110 cm connecting the patient having a catheter to the top of the first chamber (4) of the liquid recipient (2), a drainage pipe (8) made of stiff plastic polymer connected to the external valve (6), two commercial capacitive sensors (11, 12) for continuous measurement of liquid level without contact with the fluid, an electronic unit (9) and, linked to it, a calculation unit (10). The liquid recipient (2) is a sterile commercial urinometer, model Unometer 500 manufactured in styrene acrylonitrile such that the first chamber (4) of 40 $cm^3$ is contained within the second chamber (5), which has a capacity of 500 $cm^3$, and these chambers communicate via two openings (3) located at the top of the first chamber (4). The urinometer also has an upper opening with a filter for equalizing the internal pressure with the external pressure with no risk of bacterial contamination, tabs for attaching it to the support (1) and a 2-liter urine collection bag (19) made of flexible plastic polymer connected to the drainage pipe (8). The capacitive sensors (11, 12) used are of the CLC Series from SensorTechnics, featuring a resolution of 6 bits and an average response time of 50 ms, and are attached to the external front wall of the liquid recipient (2), so that the longitudinal area of the first capacitive sensor (11) spans the full height of the first chamber (4) and the longitudinal area of the second capacitive sensor (12) spans the full height of the second chamber (5) and both the first capacitive sensor (11) and the second capacitive sensor (12) are connected to the electronic unit (9), to which they continuously send the generated voltage signal indicative of the level of liquid contained in each of the chambers of the liquid recipient (2). The electronic unit (9) has an interface for converting the TTL levels from the first CLC capacitive sensor (11) from SensorTechnics and from the second capacitive sensor (12) into TIA/EIA-232-F levels, thereby allowing serial transmission of measurements to the calculation unit (10). A personal computer is used as calculation unit (10), which allows storing the urine flow values measured in their respective time intervals.

The method for automatically measuring the amount of urine excreted by the patient is characterized in that, initially, the connection of the flexible tube (7) to the patient that has a catheter and who is in the intensive care unit is carried out. Next, the Unometer 500 is selected in the calculation unit (10) as the liquid recipient (2) to be used. With this information the calculation unit (10) adjusts the measurement range of the first capacitive sensor (11) from 0 $cm^3$ to 40 $cm^3$, the measurement range of the second capacitive sensor (12) from 40 $cm^3$ to 500 $cm^3$, $R_1$ = 40 $cm^3$ and $R_2$ = 500 $cm^3$. Consecutively, a cycle begins in which the measurement $i$ of the volume of liquid $V_i$ contained in the first chamber (4) of the liquid recipient (2) is performed with a time interval of 2 minutes, by using the first capacitive sensor (11) and the electronic unit (9). The value $V_i$ is then transmitted from the electronic unit (9) to the calculation unit (10), where it is stored. This transmission is performed serially RS-232C. In the calculation unit (10) the relative flow of urine that has flowed is calculated in accordance with the following formula:

$$Q_i = \frac{V_i - V_{i-1}}{T_1} \qquad \text{for } i = 1, 2, 3,...$$

Simultaneously, the calculation unit (10) is responsible for verifying that $Q_D < Q_i (t) < Q_u$. If at any time the calculation unit (10) verifies that this condition is not being met, a visual and audible warning signal is generated for the operator. These limit values are very important, since the lower limit $Q_D$ indicates that the patient is in anuria (they do not produce any urine) or in oliguria (they produce an excessively low amount of urine), and the upper limit $Q_u$ indicates that the patient is in polyuria (they produce an excessively high amount of urine), possibly triggered by excessive salt or glucose in the blood, or by a drug to which the patient is more sensitive than expected. Anuria, oliguria and polyuria all carry a potential life-threatening risk and these are situations that must be analyzed in more detail within the individual context of each patient. Hence the interest in having a device capable of monitoring their occurrence and of immediately notifying the nursing staff.

If at the time when $V_i \geq R_1 - \varepsilon$, where $\varepsilon$ is 2 $cm^3$, the total time required to have reached said volume of liquid in the first chamber (4) exceeds a predetermined value $L_1$, the external valve (6) is opened in order to empty the first chamber (4). $L_1$ is given by:

$$L_1 = \frac{0.5 \cdot P}{R_1 - \varepsilon}$$

where the constant 0.5 $cm^3$/(kg h) is the minimum production of urine per kilo of patient body weight and per hour to be within normality and $P$ is the patient's body mass. The opening of the external valve (6) is performed manually by nursing staff of the intensive care unit. The calculation unit (10) generates an audible warning to alert the nursing staff that said opening should be carried out. After the time needed for emptying the first chamber (4) has passed, the nursing staff shuts the external valve (6) and it is proceeded to a new measurement cycle.

If at the time when $V_i \geq R_1 - \varepsilon$, the total time required to have reached said volume of liquid in the first chamber (4) is equal to or less than a predetermined value $L_1$, the level of urine production is within the therapeutic objectives and the accurate monitoring of the urine production of the patient is not necessary. Therefore, and in order to avoid the need for continuous interventions of the nursing staff to open the external valve (6), the liquid will be allowed to fill the first chamber (4) and start to seep into the second chamber (5). A second cycle then begins in which the measurement $i$ of the volume of liquid $W_i$ contained in the second chamber (5) of the liquid recipient (2) is carried out with a time interval of 5 minutes, using for this the second capacitive sensor (12) and the electronic unit (9). The value $W_i$ is transmitted from the electronic unit (9) to the calculation unit (10), where it is stored. This transmission is performed serially RS-232C. Then, the relative flow of urine that has flowed is calculated in the calculation unit (10) in accordance with the following formula:

$$S_i = \frac{W_i - W_{i-1}}{T_2} \qquad \text{for } i = 1, 2, 3, \dots$$

Simultaneously, the calculation unit (10) is responsible for verifying that the calculated value $S_i(t)$ is lower than the upper limit value $S_u$, which indicates that the patient would be in polyuria, and higher than the lower limit value $S_D$, which indicates that the patient would be in anuria or oliguria, being $S_u = Q_u$ and $S_D = Q_D$. If at any time the calculation unit (10) verifies that this condition is not being met, a visual and audible warning signal is generated for the operator. The process stops at the point at which $W_i > R_2$ - **E**, where **E** is equal to 25 $cm^3$. That is, the process stops when the liquid that has flowed is sufficient to fill 95% of the capacity of the second chamber (5) of the liquid recipient (2). The process can also stop if the critical condition $S_i(t) < S_D$ occurs. Next the external valve (6) is opened manually, allowing completely emptying the liquid recipient (2) into the urine collection bag (19). After the time required for emptying the liquid recipient (2), the external valve (6) is closed manually and the first cycle begins again.

## Claims

1. Device for automatically measuring the amount of flowing liquid, which device is composed by a support (1) in which it is installed a liquid recipient (2) the interior of which is divided into two chambers that are communicating via at least one opening (3) located on the top of the chambers: a first chamber (4) of small size suitable for measurements in which a high resolution is desired and a second larger chamber (5) for measurements that do not require high resolution, a flexible tube (7) connected to a liquid source allowing the entry of liquid by the top of the first chamber (4) of the liquid recipient (2), a drainage pipe (8) connected to an external valve (6), an electronic unit (9) and linked to it, a calculation unit (10), and wherein the liquid recipient (2) has two volume sensors (11, 12) installed on the outer wall thereof so that the longitudinal area of the first sensor (11) spans the full height of the first chamber (4) and the longitudinal area of the second sensor (12) spans the full height of the second chamber (5) and both the first sensor (11) and the second sensor (12) are connected to the electronic unit (9), to which they continuously send the electric signal generated indicative of the level of liquid contained in each of the chambers of the liquid recipient (2); **characterized in that** the external valve (6) at the bottom of the liquid recipient (2) is adapted to allow evacuating simultaneously the first chamber (4) and the second chamber (5) of the liquid recipient (2), wherein the volume sensors are capacitive sensors and wherein the calculation unit is adapted to perform the following steps:

     a) the characteristics of the liquid recipient (2) to be used are selected in the calculation unit (10), so that the calculation unit (10) can not only adjust the measurement field of the first capacitive sensor (11) and of the second capacitive sensor (12) depending on the dimensions of the first chamber (4) and the second chamber (5), respectively, but it can also determine the values $R_1$ and $R_2$ corresponding to the maximum volumes of liquid that the first chamber (4) and the second chamber (5), respectively, may contain;

b) a cycle is started in which the measurement $i$ of the liquid volume $V_i$ contained in the first chamber (4) of the liquid recipient (2) with a predetermined time interval $T_1$ is carried out, using for this the first capacitive sensor (11);

c) the relative flow of the liquid that has flowed is calculated in the calculation unit (10) in accordance with the following formula:

$$Q_i = \frac{V_i - V_{i-1}}{T_1} \qquad \text{for } i = 1, 2, 3, \ldots$$

d) a warning signal is generated for the operator in the case that the condition $Q_D < Q_i\,(t) < Q_u$ is not met, where $Q_D$ and $Q_u$ are predetermined limit values;

e) if at the time when $V_i \geq R_1 - \varepsilon$, where $\varepsilon$ is a predetermined small value, the total time required to have reached said volume of liquid in the first chamber (4) exceeds a predetermined value $L_1$, it is proceeded to opening the external valve (6) for a time $T_{v1}$, during which complete emptying of the first chamber (4) occurs and, after this time, the external valve (6) is closed and return to step b);

f) if at the time when $V_i \geq R_1 - \varepsilon$, where $\varepsilon$ is a predetermined small value, the total time required to have reached said volume of liquid in the first chamber (4) is equal to or less than a predetermined value $L_1$, the liquid is allowed to overflow from the first chamber (4) into the second chamber (5) and a second cycle starts in which the measurement $i$ of the volume of liquid $W_i$ contained in the second chamber (5) of the liquid recipient (2) with a predetermined time interval $T_2$, using the second capacitive sensor (12) for this;

g) the calculation unit (10) calculates the relative flow of liquid that has flowed in accordance with the following formula:

$$S_i = \frac{W_i - W_{i-1}}{T_2} \qquad \text{for } i = 1, 2, 3, \ldots$$

h) a warning signal is generated for the operator if the condition $S_D < S_i\,(t) < S_u$ is not being met, where $S_D$ and $S_u$ are predetermined limit values;

i) the process is stopped when $W_i > R_2 - \mathbf{E}$, where $\mathbf{E}$ is a predetermined small value, or when the condition $S_i\,(t) < S_D$ occurs;

j) the external valve (6) is opened for a predetermined time $T_{V2}$ during which complete emptying of the liquid recipient (2) occurs, and after this time, the external valve (6) is closed and return to step b).

2.  Device for automatically measuring the amount of flowing liquid according to claim 1, wherein the liquid recipient (2) has on its external wall, two holders (13) made by way of sleeves, which allow installing both the first capacitive sensor (11) and the second capacitive sensor (12) by simply inserting the same inside the holders (13).

3.  Device for automatically measuring the amount of flowing liquid according to claim 1, wherein the device further contains a base (14) connected to the support (1), to which the capacitive sensors (11, 12) are attached and which allows to install and remove the liquid recipient (2).

4.  Device for automatically measuring the amount of flowing liquid according to claim 1, wherein the device further contains an adjustable and removable frame (15), which allows configuring the assembly of the capacitive sensors (11, 12) on the outer wall of the liquid recipient (2) and which is made from at least four rigid elements, the length of which can be varied (16) and the ends of which are joined by brackets (17), and the capacitive sensors (11, 12) are installed, by means of attachment elements (18), on at least one of these rigid elements, the length of which can be varied (16).

5.  Device for automatically measuring the amount of liquid flowing according to claims 1-4, wherein the external valve (6) is equipped with an actuator that is electrically connected to the electronic unit (9).

6.  Device for automatically measuring the amount of liquid flowing according to claims 1-5, wherein the device further has a liquid collection bag (19) connected to the drainage pipe (8) and located at a level lower than the liquid recipient (2) and the volume of which is *n* times higher than the volume of the liquid recipient (2), *n* being an integer greater than one.

7. Device for automatically measuring the amount of liquid flowing according to claims 1-6, wherein the connection between the electronic unit (9) and the calculation unit (10) is performed wirelessly.

8. Method for automatically measuring the amount of flowing liquid using a device according to claims 1-7 comprising the following steps:

a) the connection of the flexible tube (7) to the liquid source is carried out;

b) the characteristics of the liquid recipient (2) to be used are selected in the calculation unit (10), so that the calculation unit (10) can not only adjust the measurement field of the first capacitive sensor (11) and of the second capacitive sensor (12) depending on the dimensions of the first chamber (4) and the second chamber (5), respectively, but it can also determine the values $R_1$ and $R_2$ corresponding to the maximum volumes of liquid that the first chamber (4) and the second chamber (5), respectively, may contain;

c) a cycle is started in which the measurement *i* of the liquid volume $V_i$ contained in the first chamber (4) of the liquid recipient (2) with a predetermined time interval $T_1$ is carried out, using for this the first capacitive sensor (11);

d) the relative flow of the liquid that has flowed is calculated in the calculation unit (10) in accordance with the following formula:

$$Q_i = \frac{V_i - V_{i-1}}{T_1} \qquad \text{for } i = 1, 2, 3, \ldots$$

e) a warning signal is generated for the operator in the case that the condition $Q_D < Q_i(t) < Q_u$ is not met, where $Q_D$ and $Q_u$ are predetermined limit values;

f) if at the time when $V_i \geq R_1 - \varepsilon$, where $\varepsilon$ is a predetermined small value, the total time required to have reached said volume of liquid in the first chamber (4) exceeds a predetermined value $L_1$, it is proceeded to opening the external valve (6) for a time $T_{v1}$, during which complete emptying of the first chamber (4) occurs and, after this time, the external valve (6) is closed and return to step c);

g) if at the time when $V_i \geq R_1 - \varepsilon$, where $\varepsilon$ is a predetermined small value, the total time required to have reached said volume of liquid in the first chamber (4) is equal to or less than a predetermined value $L_1$, the liquid is allowed to overflow from the first chamber (4) into the second chamber (5) and a second cycle starts in which the measurement *i* of the volume of liquid $W_i$ contained in the second chamber (5) of the liquid recipient (2) with a predetermined time interval $T_2$, using the second capacitive sensor (12) for this;

h) the calculation unit (10) calculates the relative flow of liquid that has flowed in accordance with the following formula:

$$S_i = \frac{W_i - W_{i-1}}{T_2} \qquad \text{for } i = 1, 2, 3, \ldots$$

i) a warning signal is generated for the operator if the condition $S_D < S_i(t) < S_u$ is not being met, where $S_D$ and $S_u$ are predetermined limit values;

j) the process is stopped when $W_i > R_2 - \mathbf{E}$, where $\mathbf{E}$ is a predetermined small value, or when the condition $S_i(t) < S_D$ occurs;

k) the external valve (6) is opened for a predetermined time $T_{V2}$ during which complete emptying of the liquid recipient (2) occurs, and after this time, the external valve (6) is closed and return to step c).

**Patentansprüche**

1. Vorrichtung zur automatischen Messung der Menge einer strömenden Flüssigkeit, bestehend aus:

einem Träger (1), in den eine Flüssigkeitsaufnahmeeinrichtung (2) montiert ist, deren Inneres in zwei Kammern aufgeteilt ist, die über mindestens eine Öffnung (3), die am oberen Ende der Kammern liegt, kommunizieren:

eine erste Kammer (4) von kleiner Größe, geeignet für Messungen, bei denen eine hohe Auflösung gewünscht wird, und eine zweite größere Kammer (5) für Messungen, die keine hohe Auflösung erfordern,

einer biegsamem Röhre (7), die mit einer Flüssigkeitsquelle verbunden ist, und den Eintritt von Flüssigkeit durch das obere Ende der ersten Kammer (4) der Flüssigkeitsaufnahmeeinrichtung (2) gestattet, einer Ablaufleitung (8), die mit einem äußeren Ventil (6) verbunden ist, einer elektronische Einheit (9), und einer mit ihr verbundenen Berechnungseinheit (10), und

wobei die Flüssigkeitsaufnahmeeinrichtung (2) zwei Volumensensoren (11, 12) aufweist, die so auf der Außenseite davon montiert sind, dass die längslaufende Fläche des ersten Sensors (11) die volle Höhe der ersten Kammer (4) umspannt und die längslaufende Fläche des zweiten Sensors (12) die volle Höhe der zweiten Kammer (5) umspannt, und beide, der erste Sensor (11) und der zweite Sensor (12) mit der elektronischen Einheit (9) verbunden sind, zu der sie kontinuierlich ein erzeugtes elektrisches Signal senden, das den Stand der in jeder der Kammern der Flüssigkeitsaufnahmeeinrichtung (2) enthaltenen Flüssigkeit anzeigt;

**dadurch gekennzeichnet, dass**

das äußere Ventil (6) am Boden der Flüssigkeitsaufnahmeeinrichtung (2) geeignet ist, die gleichzeitige Entleerung der ersten Kammer (4) und der zweiten Kammer (5) der Flüssigkeitsaufnahmeeinrichtung (2) zu ermöglichen, wobei die Volumensensoren kapazitive Sensoren sind, und wobei die Berechnungseinheit geeignet ist, die nachstehenden Schritte durchzuführen:

a) die Eigenschaften der zu verwendenden Flüssigkeitsaufnahmeeinrichtung (2) werden in der Berechnungseinheit (10) so ausgewählt, dass die Berechnungseinheit (10) nicht nur das Messfeld des ersten kapazitiven Sensors (11) und des zweiten kapazitiven Sensors (12) in Abhängigkeit von den Abmessungen der ersten Kammer (4) bzw. der zweiten Kammer (5) einstellen kann, sondern auch die Werte $R_1$ und $R_2$ bestimmen kann, die den maximalen Volumina der Flüssigkeit entsprechen, die die erste Kammer (4) bzw. die zweite Kammer (5) enthalten kann;

b) ein Zyklus wird begonnen, in dem die Messung $i$ des Flüssigkeitsvolumens $V_i$, das in der ersten Kammer (4) der Flüssigkeitsaufnahmeeinrichtung (2) enthalten ist, mit einem vorgegebenen Zeitintervall $T_1$ ausgeführt wird, wofür der erste kapazitive Sensor (11) eingesetzt wird;

c) die relative Strömung der Flüssigkeit, die geflossen ist, wird in der Berechnungseinheit (10) gemäß der nachstehenden Formel berechnet:

$$Q_i = \frac{V_i - V_{i\text{-}1}}{T_1} \qquad \text{für } i = 1, 2, 3,$$

d) ein Warnsignal für den Operator wird in dem Fall erzeugt, dass die Bedingung $Q_D < Q_i\,(t) < Q_u$ nicht erfüllt wird, wobei $Q_D$ und $Q_u$ vorgegebene Grenzwerte sind;

e) wenn zu dem Zeitpunkt, an dem $V_i \geq R_1$ - e ist, wobei e ein vorgegebener kleiner Wert ist, die Gesamtzeitdauer, die erforderlich ist, damit dieses Volumen der Flüssigkeit in der ersten Kammer (4) erreicht wird, einen vorgegebenen Wert $L_1$ überschreitet, wird das Öffnen des äußeren Ventils (6) für eine Zeitdauer $T_{v1}$ fortgesetzt, während der es zu einer vollständigen Entleerung der ersten Kammer (4) kommt, und nach dieser Zeit wird das äußere Ventil (6) geschlossen und es wird zu Schritt b) zurückgekehrt;

f) wenn zu dem Zeitpunkt, an dem $V_i \geq R_1$ - e ist, wobei e ein vorgegebener kleiner Wert ist, die Gesamtzeitdauer, die erforderlich ist, damit dieses Volumen der Flüssigkeit in der ersten Kammer (4) erreicht wird, gleich oder kleiner als ein vorgegebener Wert $L_1$ ist, wird es der Flüssigkeit gestattet von der ersten Kammer (4) in die zweite Kammer (5) zu strömen, und es beginnt ein zweiter Zyklus, in dem eine Messung $i$ des Volumens der Flüssigkeit $W_i$, das in der zweiten Kammer (5) der Flüssigkeitsaufnahmeeinrichtung (2) enthalten ist, mit einem vorgegebenen Zeitintervall $T_2$ erfolgt, wofür der zweite kapazitive Sensor (12) verwendet wird;

g) die Berechnungseinheit (10) berechnet die relative Strömung einer Flüssigkeit, die geflossen ist, in Übereinstimmung mit der nachstehenden Formel geflossen ist:

$$S_i = \frac{W_i - W_{i\text{-}1}}{T_2} \qquad \text{für } i = 1, 2, 3,$$

h) ein Warnsignal für den Operator wird erzeugt, wenn die Bedingung $S_D < S_i\,(t) < S_u$ nicht erfüllt wird, wobei

$S_D$ und $S_u$ vorgegebene Grenzwerte sind;

i) der Prozess wird angehalten, wenn $W_i > R_2$ - **E,** wobei **E** ein vorgegebener kleiner Wert ist, oder wenn die Bedingung $S_i (t) < S_D$ auftritt;

j) das äußere Ventil (6) wird für eine vorgegebene Zeitdauer $T_{V2}$ geöffnet, während der es zu einer vollständigen Entleerung der Flüssigkeitsaufnahmeeinrichtung (2) kommt, und nach dieser Zeit wird das äußere Ventil (6) geschlossen und es wird zu Schritt b) zurückgekehrt.

2. Vorrichtung zur automatischen Messung der Menge einer strömenden Flüssigkeit nach Anspruch 1, wobei die Flüssigkeitsaufnahmeeinrichtung (2) auf ihrer Außenwand zwei Halterungen (13) aufweist, hergestellt mit Hilfe von Hülsen, die es gestatten, sowohl den ersten kapazitiven Sensor (11) als auch den zweite kapazitiven Sensor (12) durch einfaches Einfügen derselben ins Innere der Halterungen (13) zu installieren.

3. Vorrichtung zur automatischen Messung der Menge einer strömenden Flüssigkeit nach Anspruch 1, wobei die Vorrichtung des Weiteren einen mit dem Träger (1) verbundenen Sockel (14) enthält, an dem die kapazitiven Sensoren (11, 12) befestigt sind, und der es gestattet, die Flüssigkeitsaufnahmeeinrichtung (2) zu montieren und zu entfernen.

4. Vorrichtung zur automatischen Messung der Menge einer strömenden Flüssigkeit nach Anspruch 1, wobei die Vorrichtung des Weiteren einen einstellbaren und entfernbaren Rahmen (15) enthält, der die Gestaltung der Anordnung der kapazitiven Sensoren (11, 12) auf der Außenwand der Flüssigkeitsaufnahmeeinrichtung (2) gestattet, und aus mindestens vier starren Elementen gefertigt ist, deren Länge verändert werden kann (16) und deren Enden durch Winkel (17) verbunden sind, und wobei die kapazitiven Sensoren (11, 12) mittels Befestigungselementen (18) auf mindestens eines dieser starren Elemente, deren Länge verändert werden kann (16), montiert sind.

5. Vorrichtung zur automatischen Messung der Menge einer strömenden Flüssigkeit nach den Ansprüchen 1 bis 4, wobei das äußere Ventil (6) mit einem Aktuator ausgerüstet ist, der elektrisch mit der elektronischen Einheit (9) verbunden ist.

6. Vorrichtung zur automatischen Messung der Menge einer strömenden Flüssigkeit nach den Ansprüchen 1 bis 5, wobei die Vorrichtung des Weiteren einen Flüssigkeitssammelbeutel (19) aufweist, der mit der Ablaufleitung (8) verbunden ist und auf einer niedrigeren Höhe als die Flüssigkeitsaufnahmeeinrichtung (2) liegt, und dessen Volumen n-mal größer als das Volumen der Flüssigkeitsaufnahmeeinrichtung (2) ist, wobei n eine ganze Zahl größer als Eins ist.

7. Vorrichtung zur automatischen Messung der Menge einer strömenden Flüssigkeit nach den Ansprüchen 1 bis 6, wobei die Verbindung zwischen der elektronischen Einheit (9) und der Berechnungseinheit (10) drahtlos erfolgt.

8. Verfahren zur automatischen Messung der Menge einer strömenden Flüssigkeit unter Verwendung einer Vorrichtung nach den Ansprüchen 1 bis 7, das die nachstehenden Schritte umfasst:

a) eine Anbindung der biegsamen Röhre (7) an die Flüssigkeitsquelle wird durchgeführt;

b) die Eigenschaften der zu verwendenden Flüssigkeitsaufnahmeeinrichtung (2) werden in der Berechnungseinheit (10) so ausgewählt, dass die Berechnungseinheit (10) nicht nur das Messfeld des ersten kapazitiven Sensors (11) und des zweiten kapazitiven Sensors (12) in Abhängigkeit von den Abmessungen der ersten Kammer (4) bzw. der zweiten Kammer (5) einstellen kann, sondern auch die Werte $R_1$ und $R_2$ bestimmen kann, die den maximalen Volumina der Flüssigkeit entsprechen, die die erste Kammer (4) bzw. die zweite Kammer (5) enthalten kann;

c) ein Zyklus wird begonnen, in dem die Messung $i$ des Flüssigkeitsvolumens $V_i$, das in der ersten Kammer (4) der Flüssigkeitsaufnahmeeinrichtung (2) enthalten ist, mit einem vorgegebenen Zeitintervall $T_1$ ausgeführt wird, wofür der erste kapazitive Sensor (11) eingesetzt wird;

c) die relative Strömung der Flüssigkeit, die geflossen ist, wird in der Berechnungseinheit (10) gemäß der nachstehenden Formel berechnet:

$$Q_i = \frac{V_i - V_{i-1}}{T_1} \qquad \text{für } i = 1,2,3,$$

e) ein Warnsignals für den Operator wird in dem Fall erzeugt, dass die Bedingung $Q_D < Q_i(t) < Q_u$ nicht erfüllt wird, wobei $Q_D$ und $Q_u$ vorgegebene Grenzwerte sind;

f) wenn zu dem Zeitpunkt, an dem $V_i \geq R_1 - \varepsilon$ ist, wobei e ein vorgegebener kleiner Wert ist, die Gesamtzeitdauer, die erforderlich ist, damit dieses Volumen der Flüssigkeit in der ersten Kammer (4) erreicht wird, einen vorgegebenen Wert $L_1$ überschreitet, wird das Öffnen des äußeren Ventils (6) für eine Zeitdauer $T_{v1}$ fortgesetzt, während der es zu einer vollständigen Entleerung der ersten Kammer (4) kommt, und nach dieser Zeit wird das äußere Ventil (6) geschlossen und es wird zu Schritt c) zurückgekehrt;

g) wenn zu dem Zeitpunkt, an dem $V_i \geq R_1 - \varepsilon$ ist, wobei e ein vorgegebener kleiner Wert ist, die Gesamtzeitdauer, die erforderlich ist, damit dieses Volumen der Flüssigkeit in der ersten Kammer (4) erreicht wird, gleich oder kleiner als ein vorgegebener Wert $L_1$ ist, wird es der Flüssigkeit gestattet von der ersten Kammer (4) in die zweite Kammer (5) zu strömen, und es beginnt ein zweiter Zyklus, in dem die Messung $i$ des Volumens der Flüssigkeit $Wi$, das in der zweiten Kammer (5) der Flüssigkeitsaufnahmeinrichtung (2) enthalten ist, mit einem vorgegebenen Zeitintervall $T_2$, erfolgt, wofür der zweite kapazitive Sensor (12) verwendet wird;

h) die Berechnungseinheit (10) berechnet die relative Strömung einer Flüssigkeit, die geflossen ist, gemäß der nachstehenden Formel:

$$S_i = \frac{W_i - W_{i-1}}{T_2} \qquad \text{für } i = 1, 2, 3,$$

i) ein Warnsignal wird für den Operator erzeugt, wenn die Bedingung $S_D < S_i(t) < S_u$ nicht erfüllt wird, wobei $S_D$ und $S_u$ vorgegebene Grenzwerte sind;

j) der Prozess wird angehalten, wenn $W_i > R_2 - \mathbf{E}$, wobei $\mathbf{E}$ ein vorgegebener kleiner Wert ist, oder wenn die Bedingung $S_i (t) < S_D$ auftritt;

k) das äußere Ventil (6) wird für eine vorgegebene Zeitdauer $T_{V2}$ geöffnet, während der es zu einer vollständigen Entleerung der Flüssigkeitsaufnahmeinrichtung (2) kommt, und nach dieser Zeit wird das äußere Ventil (6) geschlossen und es wird zu Schritt c) zurückgekehrt.

## Revendications

1. Dispositif de mesure automatique de la quantité de liquide s'écoulant, lequel dispositif est composé par un support (1) dans lequel est installé un récipient de liquide (2) dont l'intérieur est divisé en deux chambres qui communiquent par l'intermédiaire d'au moins une ouverture (3) située sur la partie supérieure des chambres : une première chambre (4) de petite taille pertinente pour des mesures dans lesquelles une résolution élevée est souhaitée, et une seconde chambre plus grande (5) pour des mesures qui ne nécessitent pas de résolution élevée, un tube flexible (7) raccordé à une source de liquide permettant l'entrée de liquide par la partie supérieure de la première chambre (4) du récipient de liquide (2), un tuyau de drainage (8) raccordé à une vanne externe (6), une unité électronique (9), et reliée à celle-ci, une unité de calcul (10), et dans lequel le récipient de liquide (2) présente deux capteurs de volume (11, 12) installés sur la paroi externe de celui-ci, de sorte que la zone longitudinale du premier capteur (11) s'étend sur la pleine hauteur de la première chambre (4) et la zone longitudinale du second capteur (12) s'étend sur la pleine hauteur de la seconde chambre (5), et à la fois le premier capteur (11) et le second capteur (12) sont connectés à l'unité électronique (9), à laquelle ils envoient en permanence le signal électrique généré indicatif du niveau de liquide contenu dans chacune des chambres du récipient de liquide (2) ; **caractérisé en ce que** la vanne externe (6) dans la partie inférieure du récipient de liquide (2) est apte à permettre d'évacuer simultanément la première chambre (4) et la seconde chambre (5) du récipient de liquide (2), dans lequel les capteurs de volume sont des capteurs capacitifs et dans lequel l'unité de calcul est apte à mettre en oeuvre les étapes ci-dessous dans lesquelles :

a) les caractéristiques du récipient de liquide (2) à utiliser sont sélectionnées dans l'unité de calcul (10), de sorte que l'unité de calcul (10) peut non seulement ajuster le champ de mesure du premier capteur capacitif (11) et du second capteur capacitif (12) en fonction des dimensions de la première chambre (4) et de la seconde chambre (5), respectivement, mais qu'elle peut également déterminer les valeurs $R_1$ et $R_2$ correspondant aux volumes maximaux de liquide que la première chambre (4) et la seconde chambre (5), respectivement, peuvent contenir ;

b) un cycle est démarré dans lequel la mesure $i$ du volume de liquide $V_i$ contenu dans la première chambre (4) du récipient de liquide (2), avec un intervalle de temps prédéterminé $T_1$, est effectuée, en utilisant pour cela le premier capteur capacitif (11) ;

c) l'écoulement relatif du liquide qui s'est écoulé est calculé dans l'unité de calcul (10) selon la formule suivante :

$$Q_i = \frac{V_i - V_{i-1}}{T_1}$$

pour $i$ = 1, 2, 3,

d) un signal d'avertissement est généré pour l'opérateur dans le cas où la condition $Q_D < Q_i\,(t) < Q_u$ n'est pas satisfaite, où $Q_D$ et $Q_u$ sont des valeurs limites prédéterminées ;

e) si, à l'instant où $V_i \geq R_1 - \varepsilon$, où $\varepsilon$ est une petite valeur prédéterminée, le temps total requis pour atteindre ledit volume de liquide dans la première chambre (4) est supérieur à une valeur prédéterminée $L_1$, il est procédé à l'ouverture de la vanne externe (6) pour un temps $T_{V1}$, au cours duquel une vidange complète de la première chambre (4) se produit, et passé ce délai, la vanne externe (6) est fermée et le processus revient à l'étape b) ;

f) si, à l'instant où $V_i \geq R_1 - \varepsilon$, où $\varepsilon$ est une petite valeur prédéterminée, le temps total requis pour atteindre ledit volume de liquide dans la première chambre (4) est égal ou inférieur à une valeur prédéterminée $L_1$, le liquide est autorisé à déborder de la première chambre (4) dans la seconde chambre (5) et un second cycle démarre dans lequel est effectuée la mesure $i$ du volume de liquide $W_i$ contenu dans la seconde chambre (5) du récipient de liquide (2), avec un intervalle de temps prédéterminé $T_2$, en utilisant le second capteur capacitif (12) à cet effet ;

g) l'unité de calcul (10) calcule l'écoulement relatif de liquide qui s'est écoulé, selon la formule suivante :

$$S_i = \frac{W_i - W_{i-1}}{T_2}$$

pour $i$ = 1, 2, 3,

h) un signal d'avertissement est généré pour l'opérateur, si la condition $S_D < S_i\,(t) < S_u$ n'est pas satisfaite, où $S_D$ et $S_u$ sont des valeurs limites prédéterminées ;

i) le processus est interrompu lorsque $W_i > R_2 - $ **E,** où **E** est une petite valeur prédéterminée, ou lorsque la condition $S_i\,(t) < S_D$ se produit ;

j) la vanne externe (6) est ouverte pour un temps prédéterminé $T_{V2}$ au cours duquel une vidange complète du récipient de liquide (2) se produit, et passé ce délai, la vanne externe (6) est fermée, et le processus revient à l'étape b).

2. Dispositif de mesure automatique de la quantité de liquide s'écoulant selon la revendication 1, dans lequel le récipient de liquide (2) présente, sur sa paroi externe, deux éléments de retenue (13) réalisés au moyen de manchons, lesquels permettent l'installation du premier capteur capacitif (11) et du second capteur capacitif (12) en les insérant simplement à l'intérieur des éléments de retenue (13).

3. Dispositif de mesure automatique de la quantité de liquide s'écoulant selon la revendication 1, dans lequel le dispositif contient en outre une base (14) raccordée au support (1), à laquelle sont fixés les capteurs capacitifs (11, 12) et qui permet d'installer et d'enlever le récipient de liquide (2).

4. Dispositif de mesure automatique de la quantité de liquide s'écoulant selon la revendication 1, dans lequel le dispositif contient en outre un cadre ajustable et amovible (15), qui permet de configurer l'assemblage des capteurs capacitifs (11, 12) sur la paroi externe du récipient de liquide (2) et qui est réalisé à partir d'au moins quatre éléments rigides, dont la longueur peut être modifiée (16) et dont les extrémités sont jointes par des fixations (17), et dans lequel les capteurs capacitifs (11, 12) sont installés, au moyen d'éléments de fixation (18), sur au moins l'un de ces éléments rigides, dont la longueur peut être modifiée (16).

5. Dispositif de mesure automatique de la quantité de liquide s'écoulant selon les revendications 1 à 4, dans lequel la vanne externe (6) est équipée d'un actionneur qui est connecté électriquement à l'unité électronique (9).

6. Dispositif de mesure automatique de la quantité de liquide s'écoulant selon les revendications 1 à 5, dans lequel le dispositif présente en outre un sac de collecte de liquide (19) raccordé au tuyau de drainage (8) et situé à un niveau inférieur à celui du récipient de liquide (2) et dont le volume est $n$ fois plus élevé que le volume du récipient de liquide (2), $n$ étant un nombre entier supérieur à 1.

7. Dispositif de mesure automatique de la quantité de liquide s'écoulant selon les revendications 1 à 6, dans lequel la connexion entre l'unité électronique (9) et l'unité de calcul (10) est mise en oeuvre par voie hertzienne.

8. Procédé de mesure automatique de la quantité de liquide s'écoulant utilisant un dispositif selon les revendications 1 à 7, comprenant les étapes ci-dessous dans lesquelles :

a) le raccordement du tube flexible (7) à la source de liquide est mis en oeuvre ;

b) les caractéristiques du récipient de liquide (2) à utiliser sont sélectionnées dans l'unité de calcul (10), de sorte que l'unité de calcul (10) peut non seulement ajuster le champ de mesure du premier capteur capacitif (11) et du second capteur capacitif (12) en fonction des dimensions de la première chambre (4) et de la seconde chambre (5), respectivement, mais qu'elle peut également déterminer les valeurs $R_1$ et $R_2$ correspondant aux volumes maximaux de liquide que la première chambre (4) et la seconde chambre (5), respectivement, peuvent contenir ;

c) un cycle est démarré dans lequel la mesure $i$ du volume de liquide $V_i$ contenu dans la première chambre (4) du récipient de liquide (2), avec un intervalle de temps prédéterminé $T_1$, est effectuée, en utilisant pour cela le premier capteur capacitif (11) ;

d) l'écoulement relatif du liquide qui s'est écoulé est calculé dans l'unité de calcul (10) selon la formule suivante :

$$Q_i = \frac{V_i - V_{i-1}}{T_1} \qquad \text{pour } i = 1, 2, 3,$$

e) un signal d'avertissement est généré pour l'opérateur dans le cas où la condition $Q_D < Q_i(t) < Q_u$ n'est pas satisfaite, où $Q_D$ et $Q_u$ sont des valeurs limites prédéterminées ;

f) si, à l'instant où $V_i \geq R_1 - \varepsilon$, où $\varepsilon$ est une petite valeur prédéterminée, le temps total requis pour atteindre ledit volume de liquide dans la première chambre (4) est supérieur à une valeur prédéterminée $L_1$, il est procédé à l'ouverture de la vanne externe (6) pour un temps $T_{V1}$, au cours duquel une vidange complète de la première chambre (4) se produit, et passé ce délai, la vanne externe (6) est fermée et le processus revient à l'étape c) ;

g) si, à l'instant où $V_i \geq R_1 - \varepsilon$, où $\varepsilon$ est une petite valeur prédéterminée, le temps total requis pour atteindre ledit volume de liquide dans la première chambre (4) est égal ou inférieur à une valeur prédéterminée $L_1$, le liquide est autorisé à déborder de la première chambre (4) dans la seconde chambre (5) et un second cycle démarre dans lequel est effectuée la mesure $i$ du volume de liquide $W_i$ contenu dans la seconde chambre (5) du récipient de liquide (2), avec un intervalle de temps prédéterminé $T_2$, en utilisant le second capteur capacitif (12) à cet effet ;

h) l'unité de calcul (10) calcule l'écoulement relatif de liquide qui s'est écoulé, selon la formule suivante :

$$S_i = \frac{W_i - W_{i-1}}{T_2} \qquad \text{pour } i = 1, 2, 3,$$

i) un signal d'avertissement est généré pour l'opérateur, si la condition $S_D < S_i (t) < S_u$ n'est pas satisfaite, où $S_D$ et $S_u$ sont des valeurs limites prédéterminées ;

j) le processus est interrompu lorsque $W_i > R_2 - \mathbf{E}$, où $\mathbf{E}$ est une petite valeur prédéterminée, ou lorsque la condition $S_i (t) < S_D$ se produit ;

k) la vanne externe (6) est ouverte pour un temps prédéterminé $T_{V2}$ au cours duquel une vidange complète du récipient de liquide (2) se produit, et passé ce délai, la vanne externe (6) est fermée, et le processus revient à l'étape c).

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0008450 A
- EP 0471413 A
- DE 3240191
- DE 4023336
- US 4745929 A
- DE 3544031
- DE 4338687

### Non-patent literature cited in the description

- **A. OTERO.** A low cost device for monitoring the urine output of critical care patients. *SENSORS,* 02 December 2010, vol. 10 (12), ISSN 1424-8220, 10714-10732
- A Prototype Device to Measure and Supervise Urine Output of Critical Patients. **A. OTERO.** Recent Advances in Biomedical Engineering. InTech, 01 October 2009, 321-334